# EUROPEAN PATENT APPLICATION

(11) **EP 1 470 776 A2**
(43) Date of publication of application: **27.10.2004**
(21) Application number: 04009392.4
(22) Date of filing: 21.04.2004
(51) Int. Cl.: A47K 7/00

(54) **Microdermabrasive exfoliator**

(30) Priority: 24.04.2003 US 422246
(71) Applicant: Trimar Visions, LLC, Delray Beach, FL 33483 (US)
(72) Inventor: Winitsky, Kathleen M., Delray Beach Florida 33444 (US)
(74) Representative: Zambardino, Umberto

(57) **Abstract**

The present invention concerns a microdermabrasive exfoliator including a porous pad and a porous microdermabrasive layer containing a plurality of microdermabrasive particulates affixed to at least a portion of a top surface of the porous pad. The porous microdermabrasive layer further includes a porous adhesive layer in which the particulates can be affixed to the portion of the top surface of the porous pad with the porous adhesive layer. In one arrangement, the microdermabrasive particulates can be aluminum oxide particulates or silicon carbide particulates.

## Description

### Field of Application

The present invention relates generally to cosmetics and more particularly, to skin exfoliators for removing dead skin cells.

### Prior Art

Currently, millions of people use a wide variety of products and procedures to improve the appearance of their skin. For example, personal hygiene products such as alpha-hydroxy acids and facial scrubs enable a person to exfoliate their skin. This exfoliation process enables a person to remove dead skin cells. Although products such as those listed above are generally sufficient, treatments that are more effective are available. One such treatment is microdermabrasion. In microdermabrasion, micro-crystals are vacuumed through a hand piece and directed at an angle onto an area of a patient's skin. Using microdermabrasion, a skin-care technician can effect a superficial skin polishing.

Microdermabrasion treatments are known to be lengthy and expensive sessions. For example, a single microdermabrasion session typically can last more than thirty minutes and cost more than $90. As a result, what is needed in the art is a simple, cost-effective solution for performing skin exfoliation.

### Summary of the Invention

The present invention concerns a microdermabrasive exfoliator. The microdermabrasive exfoliator includes a porous pad and a porous microdermabrasive layer containing a plurality of microdermabrasive particulates affixed to at least a portion of a top surface of the porous pad. In one arrangement, the porous microdermabrasive layer can further include a porous adhesive layer in which the particulates can be affixed to the portion of the top surface of the porous pad with the porous adhesive layer. As an example, the microdermabrasive particulates can be aluminum oxide particulates or silicon carbide particulates.

The present invention also concerns a microdermabrasive exfoliator including a porous pad and a microdermabrasive layer having an adhesive layer and a plurality of microdermabrasive particulates. The adhesive layer affixes the microdermabrasive particulates to a top surface of the porous pad in which the microdermabrasive particulates are at least one of aluminum oxide particulates and silicon carbide particulates. In another arrangement, the microdermabrasive particulates are smaller than 90 microns in size.

### Brief Description of the drawings

FIG. 1 illustrates a microdermabrasive exfoliator in accordance with the inventive arrangements.
FIG. 2 illustrates an enlarged view of the microdermabrasive exfoliator of FIG. 1 in accordance with the inventive arrangements.
FIG. 3 illustrates another enlarged view of the microdermabrasive exfoliator of FIG. 1 in accordance with the inventive arrangements.
FIG. 4 illustrates an example of another microdermabrasive exfoliator in accordance with the inventive arrangements.

### Detailed Description of the Preferred Embodiments

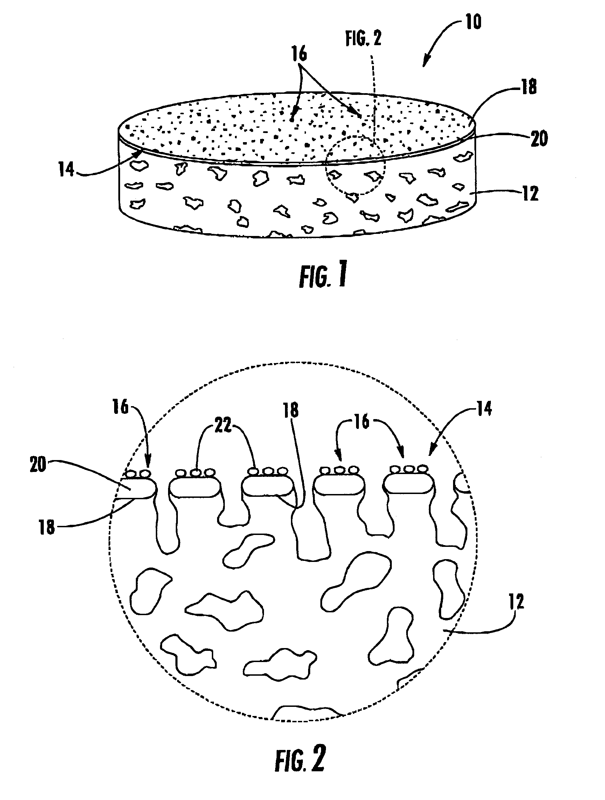

Referring to FIG. 1, a microdermabrasive exfoliator 10 in accordance with the inventive arrangements is shown. The microdermabrasive exfoliator 10 can include a pad 12 and a microdermabrasive layer 14 containing a plurality of microdermabrasive particulates 16 affixed to at least a portion of a top surface 18 of the pad 12. In one arrangement, the pad 12 can be a porous pad, such as a sponge or a foam pad. Additionally, the microdermabrasive layer 14 can be porous. For example, the microdermabrasive layer 14 can include a porous adhesive layer 20 in which the microdermabrasive particulates 16 are affixed to at least a portion of the top surface 18 of the pad 12 with the porous adhesive layer 20.

Referring to FIG. 2, an enlarged view of a portion of the porous pad 12 and the porous microdermabrasive layer 14 is shown. As shown, the microdermabrasive particulates 16 can be affixed to the top surface 18 of the porous pad 12 by the porous adhesive layer 20. Using a porous pad 12 and a porous microdermabrasive layer 14 can assist in the removal of heat and dead skin from a user's skin when that user exfoliates his or her body with the microdermabrasive exfoliator 10.

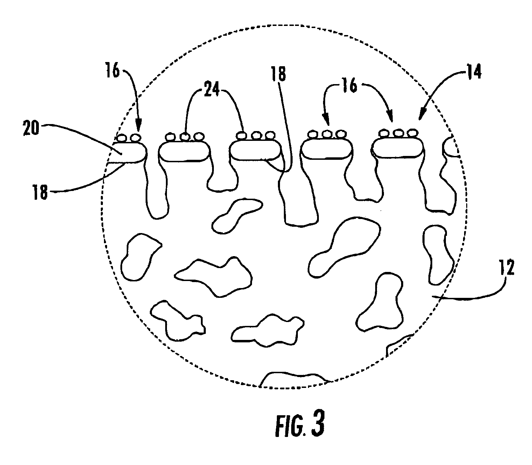

As also illustrated in FIG. 2, the microdermabrasive particulates 16 can be aluminum oxide particulates 22. Referring to FIG. 3, the microdermabrasive particulates 16 also can be silicon carbide particulates 24, or any other particulates suitable for use as a microdermabrasive. In another arrangement, the microdermabrasive particulates 16, regardless of material they are comprised of, can be smaller than 90 microns in size. For purposes of the invention, the term "size" can include the height, length, width, diameter or any other suitable dimension of the microdermabrasive particulates 16.

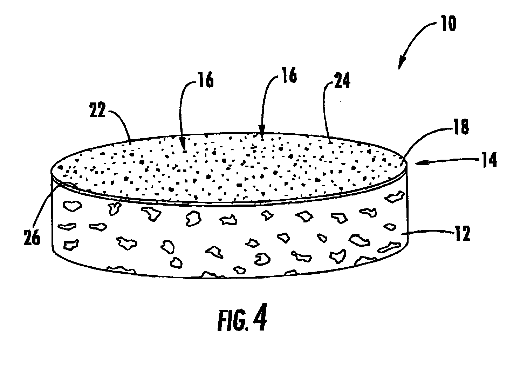

Referring to FIG. 4, another example of a microdermabrasive exfoliator 10 in accordance with the inventive arrangements is shown. In this arrangement, the microdermabrasive exfoliator 10 can include a pad 12 and a microdermabrasive layer 14 having an adhesive layer 26 and a plurality of microdermabrasive particulates 16. For example, a portion of the microdermabrasive particulates 16 can be aluminum oxide particulates 22 and another portion of the microdermabrasive particulates 16 can be silicon carbide particulates 24. Still, the invention is not so limited and any combination of suitable particulates can be used for the microdermabrasive particulates 16. In one arrangement, the microdermabrasive particulates 16 can be smaller than 90 microns in size. The adhesive layer 26 can affix the microdermabrasive particulates 16 to the top surface 18 of the pad 12. The adhesive layer 26 also can be porous or non-porous. Further, the pad 12 can be porous or non-porous.

While the preferred embodiments of the invention have been illustrated and described, it will be clear that the invention is not so limited. Numerous modifications, changes, variations, substitutions and equivalents will occur to those skilled in the art without departing from the spirit and scope of the present invention as described in the claims.

## Claims

1. A microdermabrasive exfoliator, comprising:
a porous pad; and
a porous microdermabrasive layer containing a plurality of microdermabrasive particulates affixed to at least a portion of a top surface of said porous pad.

2. The microdermabrasive exfoliator according to claim 1, wherein said porous microdermabrasive layer further comprises a porous adhesive layer, wherein said particulates are affixed to said portion of said top surface of said porous pad with said porous adhesive layer.

3. The microdermabrasive exfoliator according to claim 1, wherein said microdermabrasive particulates are aluminum oxide particulates.

4. The microdermabrasive exfoliator according to claim 1, wherein said microdermabrasive particulates are silicon carbide particulates.

5. A microdermabrasive exfoliator, comprising:
a porous pad;
a microdermabrasive layer having an adhesive layer and a plurality of microdermabrasive particulates, wherein said adhesive layer affixes said microdermabrasive particulates to a top surface of said porous pad, wherein said microdermabrasive particulates are at least one of aluminum oxide particulates and silicon carbide particulates.

6. A microdermabrasive exfoliator, comprising:
a porous pad;
a microdermabrasive layer having an adhesive layer and a plurality of microdermabrasive particulates, wherein said adhesive layer affixes said microdermabrasive particulates to a top surface of said porous pad, wherein said microdermabrasive particulates are smaller than 90 microns in size.
